# EUROPEAN PATENT SPECIFICATION

(11) **EP 0 453 216 B1**
(45) Date of publication and mention of the grant of the patent: **02.08.2000**
(21) Application number: 91303320.5
(22) Date of filing: 16.04.1991
(51) Int. Cl.: C12N 15/31, A61K 39/10

(54) **The use of autologous promoters to express gene products in bordetella**
Verwendung von autologen Promotoren zur Expression von Genprodukten in Bordetella
Utilisation de promoteurs autologues pour l'expression de produits de gène chez Bordetella

(30) Priority: 18.04.1990 GB 9008746
(43) Date of publication of application: 23.10.1991
(73) Proprietor: CONNAUGHT LABORATORIES LIMITED, Willowdale Ontario M2R 3T4 (CA)
(72) Inventor: Loosmore, Sheena, Aurora, Ontario, L4G 4R4 (CA); Zealey, Gavin, Concord, Ontario, L4K 1G8 (CA); Yacoob, Reza Khayyam, Mississauga, Ontario, L5A 2S1 (CA); Klein, Michel, Willowdale, Ontario, M5A 3M2 (CA)
(74) Representative: Burford, Anthony Frederick

(56) References cited:
- EP-A- 0 322 115
- EP-A- 0 336 413
- EP-A- 0 426 059
- WO-A-90/01494
- WO-A-90/04641
- WO-A-91/09955
- SCIENCE vol. 246, no. 4929, 27 October 1989, WASHINGTON DC, US pages 497 - 500; M. PIZZA ET AL.: 'Mutants of Pertussis toxin suitable for vaccine development'
- GENE vol. 29, no. 3, September 1984, AMSTERDAM, NL pages 231 - 241; O. RAIBAUD ET AL.: 'A technique for integrating any DNA fragment into the chromosome of Escherichia coli'

## Description

### FIELD OF INVENTION

The present invention relates to a novel approach to vary the production of gene products, particularly protein antigens, particularly in species of Bordetella, by changing the promoters of the gene coding for the respective proteins and altering their level of expression.

### BACKGROUND TO THE INVENTION

The bacterial species Bordetella comprises B. pertussis, B. parapertussis, B. bronchiseptica, and B. avium. The first two microorganisms are human pathogens, while the latter two are generally restricted to non-human hosts. B. pertussis and B. parapertussis cause the disease whooping cough with the former generating more severe symptoms. The disease, or vaccination against the disease (using an inactivated whole cell vaccine), elicits antibodies agains several antigens, typically pertussis toxin (PT), filamentous haemagglutinin (FHA), agglutinogens or fimbriae and the 69kDa outer membrane protein or pertactin. These proteins represent the major immunogens that may be included, individually or in combination, in any vaccins used to protect against the disease, whether it be the inactivated whole-cell vaccine or a defined component vaccine. Therefore, the efficient expression of these antigens from the vaccine strain is crucial.

During the production of vaccine antigens by fermentation, it has been observed that FHA is secreted at approximately 7 and 10 times tge molar level of pertactin and PT, respectively. While protein structural complexity and secretion efficiencies may be important factors influencing antigen yields, the level of expression of B. pertussis antigen genes also may be influenced by the relative strength and the regulation of their respective promoters. Thus, it may be possible to optimize antigen production by substituting autologous promoters, which may either increase or decrease the yield of selected antigens. The resulting B. pertussis strains would be more economical and better immunogens to use directly in whole-cell vaccines. In addition, promoter interchange also may represent a means to enhance fermentation and downstream processing efficiencies for component vaccines by altering the kinetics of production and yields of specific antigens.

Along with other genes, the pertussis toxin operon (TOX), the FHA operon, and the pertactin gene (PRN) are all positively regulated by the Bordetella virulence regulating gene (Bvg), formerly known as VIR. The nucelotide sequences of the TOX, FHA, and PRN structural genes and their promoters have been established and the corresponding protein sequences derived (see below) . For the TOX operon, the Bvg responsive region of the promoter has been mapped to a position -170 bp from the start of transcription. The corresponding regulatory regions of the other genes have not yet been determined.

EP-A-0426059 discloses Bordetella pertussis gene promoter regions. These promoter regions are said to be suitable for constructing vectors for the regulated expression of immunogenic protein genes of B. pertussis.

EP-A-0336413 describes expression of heterologous proteins of interest under the control of the pertussis FHA and TOX promoters in Bordetella.

### SUMMARY OF THE INVENTION

In accordance with the present invention, there is provided a novel method to alter protein expression in Bordetella species by substituting the promoter of one structural gene by that of another. It is possible to increase or decrease protein expression using this strategy as well as achieving sychronicity or asynchronicity of production.

The present invention provides a strain of Bordetella, particulary, Bordetella pertussis, which has been transformed by a hybrid pertussis gene comprising a structural pertussis gene fused at an ATG start codon to a native but autologous pertussis promoter, wherein said promoter is the TOX promoter and the structural pertussis gene is the FHA or PRN gene, said promoter is the FHA promoter and the structural pertussis gene is the TOX or PRN gene, or said promoter is the PRN promoter and the structural pertussis gene is the TOX or FHA gene. This transformed strain produces, upon culture of the transformed strain, the gene product at a yield of production which is altered from the yield of production achieved by the same Bordetella strain transformed by a homologous gene comprising a structural pertussis gene fused at an ATG start codon to its own natural pertussis promoter.

Specific transformants of specific B. pertussis strains are described in the disclosure which follows, in particular those identified as B. pertussis strains Nos. 1290-4, 390-59, 590-4 and 890-49. In addition, non-transformed B. pertussis strains Nos. 390-101 and 1090-108-3, from which the FHA operon and the PRN gene respectively have been removed, form other aspects of the invention. The invention further includes the plasmids useful in effecting transformation of B. pertussis strains.

The gene products, namely the antigenic proteins, produced by culturing the transformed cells are useful in component vaccines against B. pertussis.

An aspect of the invention allows for the easier downstream separation of the resulting proteins, which makes the production of a component vaccine more economical. A further aspect of the invention allows for the preparation of a whole-cell pertussis vaccine in which, because of improved gene expression, antigenic proteins are better distributed.

The techniques described herein for expression of antigenic pertussis proteins from transformed strains of B. pertussis may be employed with other strains of Bordetella, such as B. parapertussis, B. bronchiseptica and B. avium.

Accordingly, in yet another aspect of the present invention, there is provided a method of expression of a gene product from an organism, which comprises transforming the organism with a hybrid gene as defined above, and culturing the transformed organism to effect expression of the gene product from the transformed organism.

### BRIEF DESCRIPTION OF DRAWINGS

Figure 1 shows the sequence of the DNA cassette used to link the FHA promoter and TOX structural gene sequences, the arrow indicating the location of the ATG start codon of the S1 gene. The plasmid S-3680-10 contains the hybrid operon sandwiched between the flanking regions of TOX and was used for introduction into a TOX deleted strain of B. pertussis;
Figure 2 shows the plasmid S-3749-18 which contains the hybrid FHAp/TOX operon sandwiched between the flanking regions of FHA, used for introduction into an FHA deleted strain of B. pertussis;
Figure 3 shows the kinetics and expression levels of PT from strains (A) 10536, (B) 390-59, and (C) 590-11. Strain 390-59 contains the hybrid FHAp/TOX operon at the TOX locus while strain 590-11 contains FHAp/TOX operon at the FHA locus plus the native TOX operon at the TOX locus;
Figure 4 shows the sequence of the DNA cassette used to link the TOX promoter and FHA structural gene, the arrow indicating the ATG codon of the FHA structural gene. The plasmid S-3838-9 contains the hybrid operon sandwiched between the FHA flanking regions for delivery to the FHA locus of an FHA deleted strain;
Figure 5 shows the kinetics and expression of FHA from strains (A) 10536 and (B) 890-49 which contains the TOXp/FHA hybrid gene at the FHA locus;
Figure 6 shows the sequence of the DNA cassette used to link the FHA promoter and pertactin structural gene, the arrow indicating the ATG codon of the pertactin gene. The plasmid S-3982-7 contains the hybrid gene between the pertactin flanking regions for delivery to the pertactin locus of a PRN deleted strain;
Figure 7 shows the kinetics and expression of the 69 kDa protein from strains (A) 10536 and (B) 1290-4 which contains the FHAp/PRN hybrid at the PRN locus;
Figure 8 shows the Southern blot of restricted genomic DNA obtained from B. pertussis strains 10536 and 390-59, which contains the FHAp/TOX hybrid at the TOX locus, and indicates that the hybrid operon was correctly placed;
Figure 9 shows the Southern blot analysis of strains 10536 and 890-49, which contains the TOXp/FHA hybrid at the FHA locus, and indicates that the hybrid operon was correctly placed; and
Figure 10 shows the Southern blot analysis of strains 10536 and 1290-4, which contains the FHAp/PRN hybrid at the PRN locus, and indicates that the hybrid operon was correctly placed.

### GENERAL DESCRIPTION OF INVENTION

Bordetella pertussis 10536 is the vaccine production strain of the assignee hereof and it has been used as the initial strain for all the work detailed by the inventors herein. The genes for PT, FHA, and pertactin have been cloned and sequenced (Nicosia et al., Proc. Natl. Acad. Sci., U.S.A., 83, 4631, [1986]; Loosmore et al., Nucl. Acids Res., 17, 8365, [1989] Relman et al., Proc. Natl. Acad. Sci., U.S.A., 86, 2637, [1989]; Charles et al., Proc. Natl. Acad. Sci., U.S.A., 86, 3554, [1989]).) and the promoter regions and transcriptional start of the structural genes have been determined. The inventors have generated hybrid genes by substituting the native promoters of a given gene by promoters from another gene of the organism. This was accomplished by fusing the promoters with the structural genes at the ATG start codon of the structural gene. Such fusions result in a native but autologous promoter, and a structural gene with its natural signal sequence. The resultant hybrid genes then have been integrated at the appropriate gene loci in the chromosome of B. pertussis by homologous recombination.

As examples of the use of autologous promoters, genes have been created containing an FHA promoter with the TOX operon (FHAp/TOX), an FHA promoter with the PRN gene (FHAp/PRN), and the TOX promoter with the FHA gene (TOXp/FHA). A number of B. pertussis strains have been generated to demonstrate the efficacy of this strategy. In all cases, the autologous, promoter functioned at its new location on the genome.

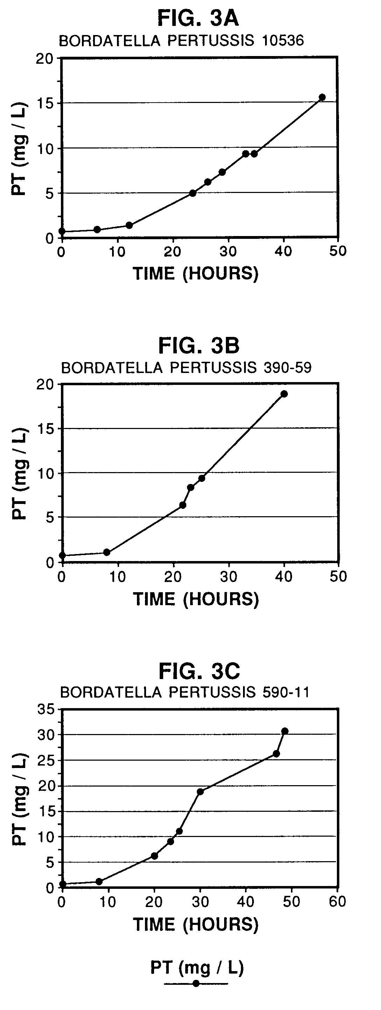

It was clearly demonstrated by this work that the promoter of a structural gene may be replaced by that of another gene, by inserting the FHAp/TOX hybrid at the TOX locus to generate B. pertussis strain 390-59. PT was secreted by this recombinant strain with kinetics and yields comparable to the wild-type strain 10536. (see Figure 3B in comparison to normal kinetics of strain 10536 shown in Figure 3A). The FHAp/TOX hybrid then was directed to the native FHA locus to generate B. pertussis strain 590-11 which now contains two copies of TOX. The yield and kinetics of PT production were consistant with the expression from two TOX genes, one directed by the FHA promoter in the hybrid operon, and the other from the native operon (see Figure 3C). This experiment demonstrated that the FHAp/TOX hybrid gene may be integrated at two different loci in the genome.

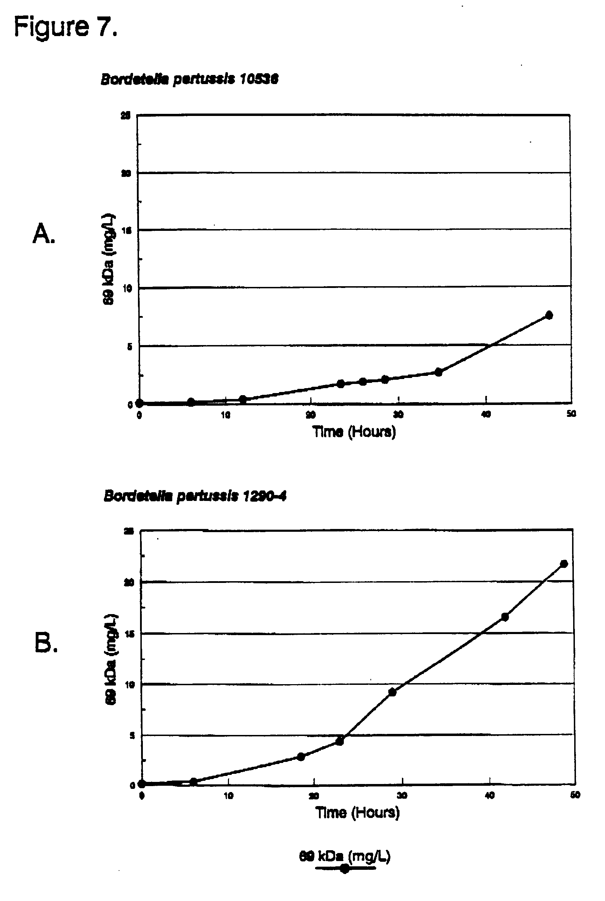

PT holotoxin is an oligomeric protein composed of six subunits encoded by five different genes. In order to determine whether the FHA promoter can direct the expression of an increased amount of protein if the protein were not as structurally complex, a strain was generated containing the FHAp/PRN hybrid gene (B. pertussis strain 1290-4). The pertactin protein is a single polypeptide produced from a larger precursor. The yield of pertactin was increased by three-fold in fermenters and ten-fold in shake flasks when its synthesis was directed by the FHA promoter (see Figure 7B, with the normal kinetics of pertactin production in a 10-litre fermenter shown in Figure 7A).

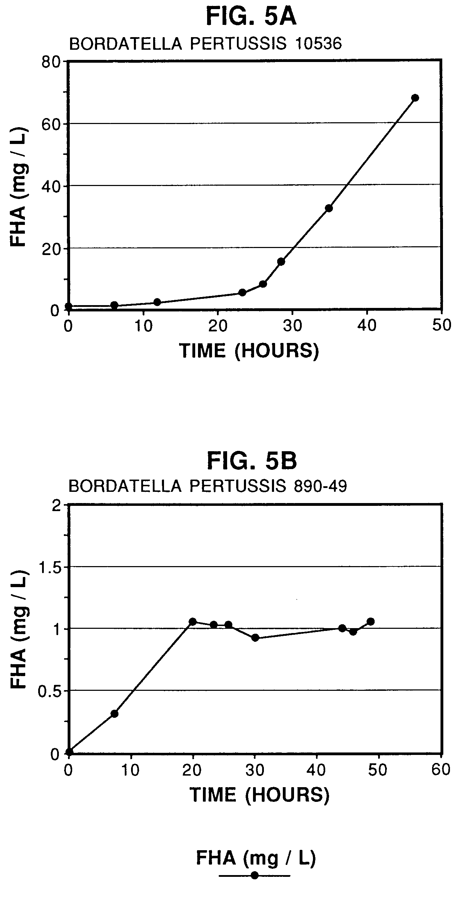

The overproduction of FHA can pose a problem in certain purification protocols. In order to reduce the yield of FHA to facilitate the purification of other antigens procuced in lesser amounts, the native promoter was substituted by the TOX promoter. The TOXp/FHA hybrid gene was directed to the FHA locus (B. pertussis strain 890-49) and the amount of FHA produced was significantly reduced, 50 to 100-fold (see Figure 5B, with normal kinetics of FHA production shown in Figure 5A).

The inventors have demonstrated by this work, as detailed in the Examples below, that it is possible to substitute promoters between two Bordetella genes and obtain antigen production from the substituted promoters. It has been further demonstrated that it is possible to increase or decrease the yield of antigens in Bordetella pertussis by promoter replacement. The production of pertactin has been increased and that of FHA decreased. Although no significant changes were observed in the kinetics of antigen production, such a promoter replacement strategy also may be used to alter the time-course as well as the yield of production of certain antigens. The regulation of protein production by this approach has broad application for fermentation and downstream processing technologies. Such a promoter replacement strategy can be used with any combination of genes in any genus of the species Bordetella and may be applied to other organisms.

### EXAMPLES

Methods of molecular genetics, fermentation, protein biochemistry, and hybridoma technology used but not explicitly described in this disclosure and these Examples are amply reported in the scientific literature and are well within the ability of those skilled in the art. Further techniques of manipulation of B. pertussis are described in published European Patent Application Publication No. 0,322,115;

All oligonucleotides were synthesized on an ABI model 380A DNA synthesizer and were purified by polycrylamide gel electrophoresis. DNA manipulations were according to Sambrook et al. (Molecular cloning: a laboratory manual, second edition, Cold Spring Harbor Laboratory, Cold Spring Harbor, N.Y. [1989]). Restriction enzymes were used according to manifacturers' specifications. The TOX, FHA, and PRN genes were cloned from a lambda Charon 35 library containing Sau3A I fragments of pertussis DNA from strain 10536. The structures of the chromosomal genes were confirmed by restriction mapping and partial or complete gene sequencing. Promoters of given genes were linked to the coding sequences of other genes using synthetic oligonucleotides. The correct fusions between the promoters and structural genes were confirmed by dideoxy DNA sequencing (Sanger et al., Proc. Natl. Acad. Sci., U.S.A. 74, 5463, [1977]). Gene replacement in B. pertussis was achieved by homologous recombination following introduction of plasmid DNA into the cell by electroporation (Zealey et al., Bio/Technology, 8, 1025, [1990]). The TOX-deleted strain 29-8, FHA-deleted strain 390-101, and PRN-deleted strain 1090-108-3 were all prepared in a similar manner. In some instances, genes were integrated as non-replicating plasmids and allelic replacement achieved by growth of primary transformants under streptomycin selection (Stibitz et al., Gene, 50, 133, [1986]). Chromosomal DNA was prepared as described by Yacoob and Zealey (Nucl. Acids Res. 16, 1639, [1988]) and Southern blot analysis performed according to Sambrook et al. using Gene Screen Plus (Dupont).

### Example 1:

This Example illustrates the construction of the FHAp/TOX hybrid operon and its introduction into B. pertussis 10536.

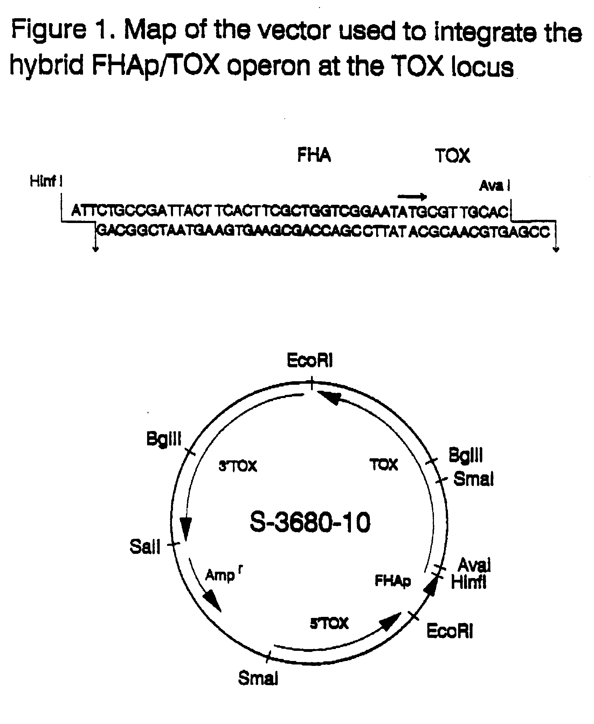

A pUC-based plasmid (S-3680-10) was constructed which contains the FHA promoter directing the expression of the native pertussis toxin structural gene and surrounded by the TOX flanking regions. The FHA promoter is an EcoR I/Hinf I fragment of approximately 240 bp which includes any Bvg responsive elements (the TOX operon requires 170 bp for the Bvg responsive region). The complimentary oligonucleotides bridging the two gene sequences were annealed as an approximately 45 bp Hinf I/Ava I cassette which includes the start codon of the DNA segment coding for the leader sequence of PT subunit S1 (see Figure 1). The remainder of the TOX operon from the Ava I site of the S1 gene to the EcoR I following the end of translation was used to complete the hybrid operon. The 3 kb Sma I/EcoR I 5′-flanking region and 4 kb EcoR I/Sal I 3′-flanking sequence of the native TOX locus were used to direct the hybrid operon to the TOX locus. (Figure 1).

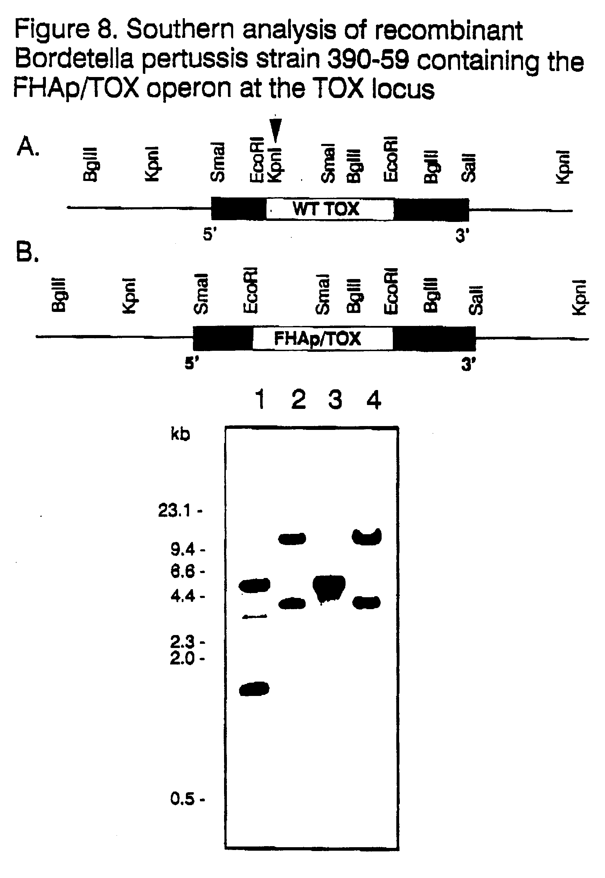

The FHAp/TOX hybrid plasmid was introduced into a TOX deleted derivative of B. pertussis 10536 (strain 29-8) by electroporation and this generated a strain (390-59) in which PT expression is directed by the FHA promoter. The kinetics and yield of PT expression from strain 390-59 were equivalent to those obtained with the parent strain 10536 (Figures 3A and 3B). The correct in situ integration of the FHAp/TOX hybrid operon at the TOX locus was demonstrated by restriction mapping and Southern blot analysis as shown in Figure 8. The TOX deleted strain 29-8 has been deposited with the ATCC (accession number 53973) and is described in the aforementioned USSN 275,376.

### Example 2:

This Example illustrates the construction of the FHAp/TOX hybrid operon and its introduction into an FHA-deleted strain of B. pertussis.

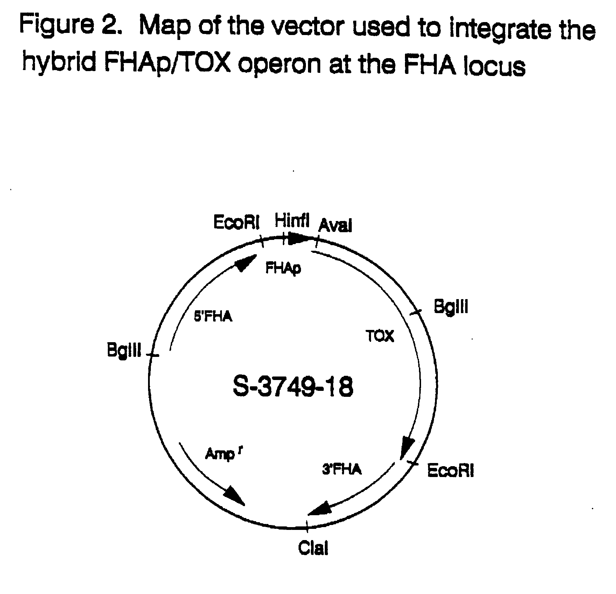

A pBR322-based plasmid (S-3749-18) was constructed which contains the FHA promoter fused to the structural genes for TOX at the start codon of the DNA segment coding for the S1 subunit leader sequence, surrounded by the FHA flanking regions. The hybrid operon was constructed as in Example 1. A 2.5 kb Bgl II/EcoR I 5′-flanking and a 1.7 kb EcoR I/Cla I 3′-flanking regions were used to direct the hybrid operon to the FHA locus (Figure 2).

This FHAp/TOX hybrid plasmid was introduced into the FHA-deleted strain (390-101). This generated a strain (590-11) with two copies of TOX, one transcribed by its native promoter and one by the FHA promoter. The overall yield of PT was about twice that of strain 10536 and the kinetics of PT production were unchanged (see Figures 3A and 3B). The FHA-deleted strain 390-101 has been deposited with the ATCC on March 6, 1991 (accession number ATCC 55157).

### Example 3:

This Example illustrates the construction of the TOXp/FHA hybrid gene and its introduction into B. pertussis.

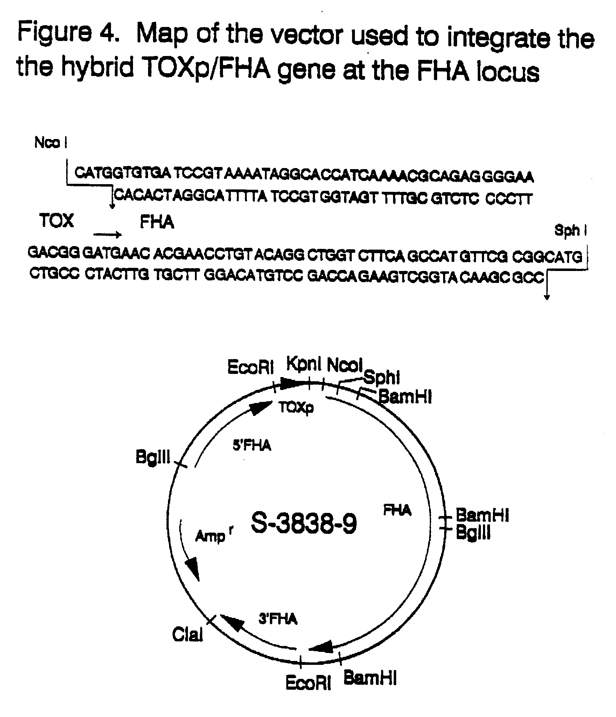

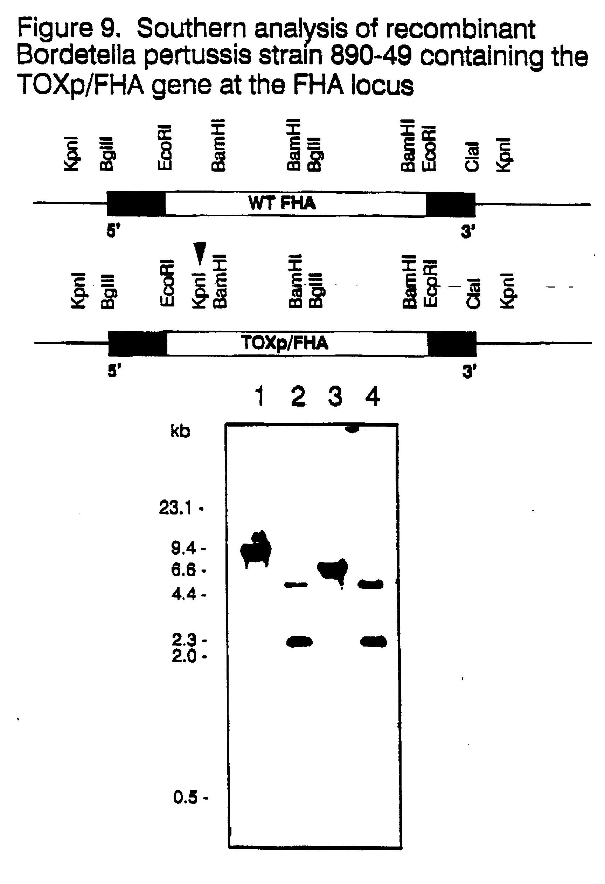

A pBR322-based plasmid (S-3838-9) was constructed which contains an approximately 500 bp of the TOX promoter region, starting at the EcoR I site. This sequence includes the Bvg responsive region at -170 bp. The 460 bp EcoR I/Nco I fragment of the TOX promoter is bridged to the FHA structural gene through an -100 bp Nco I/Sph I oligonucleotide (Figure 4). The remainder of the FHA B structural gene from the Sph I site to the EcoR I site completed the hybrid operon. The 2.5 kb Bgl II/EcoR I site FHA 5′-(flanking and 1.7 kb EcoR I/Cla I 3′-flanking regions directed the hybrid operon to the FHA locus (Figure 4). The correct integration of the TOXp/FHA hybrid operon at the FHA locus was demonstrated by restriction mapping and Southern blot analysis as shown in Figure 9.

The strain (890-49) resulting from the chromosomal integration of the hybrid operon produced significantly reduced levels of FHA (Figures 5A and 5B).

### Example 4:

This example illustrates the construction of the FHAp/PRN hybrid gene and its introduction into B. pertussis.

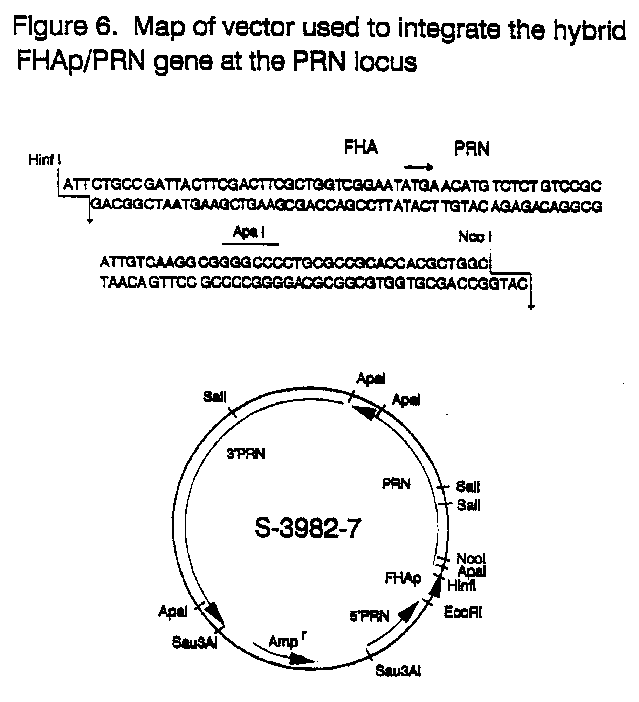

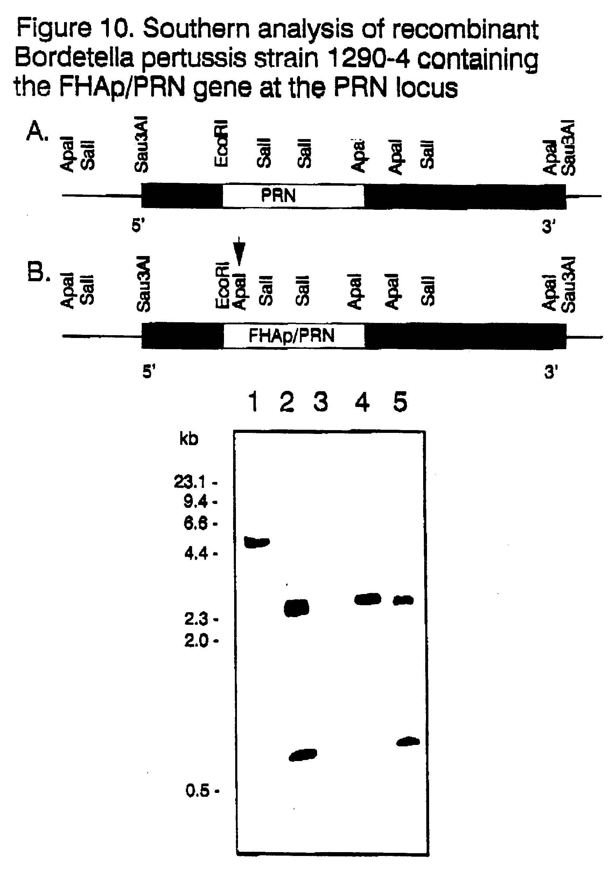

The pUC-based plasmid S-3982-7 contains the FHA promoter fused at the start codon for the signal sequence of the pertactin structural gene and surrounded by PRN flanking sequences. The FHA promoter is an approximately 240 bp EcoR I/Hinf I fragment which is fused to the PRN structural gene through a 93 bp Hinf I/Nco I oligonucleotide (Figure 6). The remainder of the pertactin structural gene from the Nco I site was added to complete the hybrid gene. The 1.6 kb Sau3A I/EcoR I 5′-flanking and 8 kb Apa I/Sau3A I 3′-flanking regions from PRN directed the hybrid gene to the PRN locus (see Figure 6). The correct in situ placement of the FHAp/PRN hybrid gene at the PRN locus of the PRN deleted strain 1090-108-3 was demonstrated by restriction mapping and Southern blot analysis in Figure 10.

Strain 1290-4 containing the FHAp/PRN hybrid produced enhanced amounts of the pertactin outer membrane protein (see Figures 7A and 7B). The PRN deleted strain 1090-108-3 and strain 1290-4 have been deposited with the ATCC on March 6, 1991 (accession numbers ATCC 55156 and ATCC 55155, respectively).

### Example 5:

This Example illustrates the preparation of B. pertussis strains containing hybrid operons and their structural analysis.

Strains deleted for TOX (29-8), FHA (390-101), or PRN (1090-108-3) were engineered using homologous recombination between the Connaught vaccine strain 10536 and plasmid DNA containing flanking regions of the desired genes, as described by Zealey et al. (Bio/Technology 8, 1025, [1990]). The flanking regions used for deleting and replacing genes were as described in Examples 1, 2, 3 and 4. The flanking regions surrounded a cassette containing a tetracycline resistance gene which was inserted at the locus for the deleted structural genes. During another round of allelic exchange, this cassette was subsequently replaced by the hybrid genes.

To confirm correct in situ placement of the hybrid genes, restriction digests and Southern blot analyses were performed on genomic DNA purified from the recombinant strains.

### Example 6:

This Example illustrates the preparation of Southern blots for the B. pertussis strain 390-59 (see Figure 8).

Chromosomal DNA was isolated from wild-type (WT) B. pertussis 10536 (lanes 1-2) and B. pertussis 390-59 (lanes 3-4), restricted with the endonucleases Kpn I/Sma I (lanes 1 and 3) and Bgl II (lanes 2 and 4) and probed with a 4.7 kb EcoR I restriction fragment that represents the entire TOX coding sequence. Replacement of the TOX promoter by the FHA promoter resulted in the loss of a KpnI site as shown by the arrow on Figure 8. Digestion with KpnI and SmaI produced TOX-specific hybridization fragments of 3.0, 1.6 and 5.1 kb for B. pertussis 10536 and 4.8 and 5.1 for B. pertussis 390-59.

### Example 7:

This Example illustrates the preparation of a Southern blot for B. pertussis strain 890-49 (Figure 9).

Chromosomal DNA was isolated from wild-type (WT) B. pertussis 10536 (lanes 1-2) and B. pertussis 890-49 (lanes 3-4), restricted with the endonucleases Kpn I/Bgl II (lanes 1 and 3) and Bam HI (lanes 2 and 4) and probed with an FHA specific probe. Replacement of the FHA promoter by the TOX promoter resulted in the introduction of a Kpn I site as shown in Figure 9. Digestion with Kpn I and Bgl II produced FHA-specific hybridizaion fragments of 9 kb for B. pertussis 10536 and 6 kb for B. pertussis 890-49.

### Example 8:

This Example illustrates the preparation of a Southern blot for B. pertussis strain 1290-4 (see Figure 10).

Chromosomal DNA was isolated from wild-type (WT) B. pertussis 10536 (lanes 1-2) and B. pertussis 1290-4 (lanes 3-4), restricted with the endonucleases Apa I (lanes 1 and 3) and Sal I (lanes 2 and 4) and probed with a PRN specific probe. Replacement of the PRN promoter by the FHA promoter resulted in the introduction of an ApaI site which produced PRN-specific hybridization fragments of 4.4 kb for B. pertussis 10536 and 2.5 kb for B. pertussis 1290-4 as shown in Figure 10.

### Example 9:

This Example illustrates the growth of B. pertussis strains and immunoasays for the quantification of specific antigens.

Bordetella pertussis strains were grown in modified Stainer-Scholte medium containing 0.2% heptakis (2,6-0-dimethyl)β-cyclodextrin (Imaizumi et al, Infect. Immun. 41, 1138, [1983]) either in 10 litre ChemAp fermenters, controlled for pH and dissolved oxygen, or in 10 ml culture. Culture supernatants were tested for antigen production in antigen-specific ELISAs. PT was measured in a fetuin-capture ELISA as described in Loosmore et al. (Infect. and Immun. 58, 3653, [1990]). FHA was captured by a mouse monoclonal anti-FHA antibody purified from ascites fluid and the second antibody was a polyclonal rabbit IgG anti-FHA conjugated to horse-radish peroxidase. Pertactin was captured with a polyclonal antibody purified from a monospecific guinea pig hyperimmune serum and the second antibody was a guinea pig IgG anti-69 kDa conjugated to horse-radish peroxidase. PT, FHA and 69 kDa proteins purified from fermentation broths of the wild-type production strain 10536 were used as standards.

### SUMMARY OF DISCLOSURE

In summary of this disclosure, the present invention provides a novel gene product expression method having particular application to Bordetella species. Modifications are possible within the scope of this invention.

## Claims

1. A strain of Bordetella characterized by being transformed by a hybrid pertussis gene comprising a structural pertussis gene fused at an ATG start codon to a native but autologous pertussis promoter, wherein said promoter is the TOX promoter and the structural pertussis gene is the FHA or PRN gene, said promoter is the FHA promoter and the structural pertussis gene is the TOX or PRN gene, or said promoter is the PRN promoter and the structural pertussis gene is the TOX or FHA gene.

2. The strain claimed in claim 1 wherein the strain of Bordetella transformed by said hybrid pertussis gene is Bordetella pertussis.

3. The strain claimed in claim 2 wherein said transformed strain produces, upon culture of said transformed strain, a gene product at a yield of production which is altered from the yield of production achieved by the same Bordetella strain containing a homologous gene comprising a structural pertussis gene fused at an ATG start codon to its own native pertussis promoter.

4. A genetically modified Bordetella strain, characterized by having the FHA gene deleted and a hybrid FHAp/TOX gene inserted into the genome to provide at least two copies of the TOX gene in the genome to effect enhanced expression levels of proteins encoded by the TOX gene present in said genetically modified strain in comparison to the natural unmodified strain.

5. The FHAp/PRN transformed PRN⁻ strain of B.pertussis No. 1290-4, as deposited with ATCC under accession No. 55155, or the FHAp/TOX (TOX) transformed TOX⁻ strain of B.pertussis No. 390-59, constructed as described in Example 1, or the FHAp/TOX (FHA) transformed FHA⁻ strain of B.pertussis No. 590-11, constructed as described in Example 2, or the TOXp/FHA (FHA) transformed FHA⁻ strain of B.pertussis No. 890-49, constructed as described in Example 3.

6. The FHA⁻ strain of Bordetella pertussis No. 390-101 having ATCC accession No. 55157, or the PRN⁻ strain of Bordetella pertussis No. 1090-108-3 having ATCC accession No. 55156.

7. Plasmid S-3680-10 comprising FHAp/TOX and TOX flanks having the restriction map shown in Figure 1, or plasmid S-3749-18 comprising FHAp/TOX and FHA flanks having the restriction map shown in Figure 2, or plasmid S-3838-9 comprising TOXp/FHA with FHA flanks having the restriction map shown in Figure 4, or plasmid S-3982-7 comprising FHAp/PRN with PRN flanks having the restriction map shown in Figure 6.

8. A whole cell vaccine against Bordetella pertussis comprising a killed form of a transformed strain of Bordetella pertussis as claimed in any one of claims 1 to 5 and a physiologically-acceptable carrier therefor.

9. A method of expression of a gene product from a transformed Bordetella strain, characterized by (a) transforming a Bordetella strain with a hybrid pertussis gene comprising a structural pertussis gene coding for a pertussis antigen fused at an ATG start codon to a native but autologous pertussis promoter, wherein said promoter is the TOX promoter and the structural pertussis gene is the FHA or PRN gene, said promoter is the FHA promoter and the structural pertussis gene is the TOX or PRN gene, or said promoter is the PRN promoter and the structural pertussis gene is the TOX or FHA gene, and (b) culturing said transformed Bordetella strain to effect expression of said pertussis antigen as said gene product from said transformed strain.

10. The method claimed in claim 9, wherein said expression of said gene product is effected at a production yield which is different from the production yield achieved wherein the Bordetella strain contains a homologous gene comprising said structural gene and its own native promoter.

11. The method claimed in claim 9 or 10, wherein transformation of said Bordetella strain is effected by integrating said hybrid gene at the appropriate gene loci in the chromosome of the Bordetella strain by homologous recombination.

12. The method claimed in any one of claims 9 to 11, wherein said Bordetella strain is Bordetella pertussis.

13. A method of expression of a gene product from a transformed Bordetella strain, which comprises culturing the Bordetella strain of claim 1.

14. The method of claim 13 wherein said Bordetella strain is Bordetella pertussis.

15. The method of claims 13 and 14 including isolating and purifying the gene product.

## Patentansprüche

1. Bordetella-Stamm, dadurch gekennzeichnet, daß er durch ein hybrides Pertussisgen, das ein Pertussis-Strukturgen, das an einem ATG-Startcodon an einen nativen, aber autologen Pertussispromotor fusioniert ist, umfaßt, transformiert ist, wobei der Promotor der TOX-Promotor ist und das Pertussis-Strukturgen das FHA- oder PRN-Gen ist, der Promotor der FHA-Promotor ist und das Pertussis-Strukturgen das TOX- oder PRN-Gen ist oder der Promotor der PRN-Promotor und das Pertussis-Strukturgen das TOX- oder FHA-Gen ist.

2. Stamm nach Anspruch 1, wobei der durch das hybride Pertussisgen transformierte Bordetella-Stamm Bordetella pertussis ist.

3. Stamm nach Anspruch 2, wobei der transformierte Stamm bei Kultur des transformierten Stamms ein Genprodukt mit einer Produktionsausbeute produziert, die gegenüber der Produktionsausbeute, die durch den gleichen Bordetella-Stamm, der ein homologes Gen, umfassend ein Pertussis-Strukturgen, das an einem ATG-Startcodon an seinen eigenen nativen Pertussispromotor fusioniert ist, enthält, erzielt wird, verändert ist.

4. Genetisch modifizierter Bordetella-Stamm, dadurch gekennzeichnet, daß in ihm das FHA-Gen deletiert und ein hybrides FHAp/TOX-Gen in das Genom insertiert ist, um mindestens zwei Kopien des TOX-Gens in dem Genom bereitzustellen, um erhöhte Expressionsniveaus von durch das TOX-Gen, das in dem genetisch modifizierten Stamm vorhanden ist, kodierten Proteinen im Vergleich zu dem natürlichen, unmodifizierten Stamm zu bewirken.

5. FHAp/PRN-transformierter PRN⁻-Stamm von B. pertussis Nr. 1290-4, wie bei der ATCC unter der Aufnahmenummer 55155 hinterlegt, oder FHAp/TOX (TOX)-transformierter TOX⁻-Stamm von B. pertussis Nr. 390-59, konstruiert, wie in Beispiel 1 beschrieben, oder FHAp/TOX (FHA)-transformierter FHA⁻-Stamm von B. pertussis Nr. 590-11, konstruiert, wie in Beispiel 2 beschrieben, oder TOXp/FHA (FHA)-transformierter FHA⁻-Stamm von B. pertussis Nr. 890-49, konstruiert, wie in Beispiel 3 beschrieben.

6. FHA⁻-Stamm von Bordetella pertussis Nr. 390-101 mit der ATCC-Aufnahmenummer 55157 oder PRN⁻-Stamm von Bordetella pertussis Nr. 1090-108-3 mit der ATCC-Aufnahmenummer 55156.

7. Plasmid S-3680-10, das FHAp/TOX und TOX-Flankenbereiche umfaßt und die in Figur 1 gezeigte Restriktionskarte aufweist, oder Plasmid S-3749-18, das FHAp/TOX und FHA-Flankenbereiche umfaßt und die in Figur 2 gezeigte Restriktionskarte aufweist, oder Plasmid S-3838-9, das TOXp/FHA mit FHA-Flankenbereichen umfaßt und die in Figur 4 gezeigte Restriktionskarte aufweist, oder Plasmid S-3982-7, das FHAp/PRN mit PRN-Flankenbereichen umfaßt und die in Figur 6 gezeigte Restriktionskarte aufweist.

8. Ganzzellimpfstoff gegen Bordetella pertussis, der eine abgetötete Form eines transformierten Stamms von Bordetella pertussis, wie in einem der Ansprüche 1 bis 5 beansprucht, und einen physiologisch annehmbaren Träger dafür umfaßt.

9. Verfahren zur Expression eines Genprodukts durch einen transformierten Bordetella-Stamm, gekennzeichnet dadurch, daß (a) ein Bordetella-Stamm mit einem hybriden Pertussisgen, das ein Pertussis-Strukturgen, das ein Pertussisantigen kodiert, fusioniert an einem ATG-Startcodon an einen nativen, aber autologen Pertussispromotor umfaßt, transformiert wird, wobei der Promotor der TOX-Promotor ist und das Pertussis-Strukturgen das FHA- oder PRN-Gen ist, der Promotor der FHA-Promotor ist und das Pertussis-Strukturgen das TOX- oder PRN-Gen ist oder der Promotor der PRN-Promotor ist und das Pertussis-Strukturgen das TOX- oder FHA-Gen ist, und (b) der transformierte Bordetella-Stamm kultiviert wird, um eine Expression des Pertussisantigens als das Genprodukt durch den transformierten Stamm zu bewirken.

10. Verfahren nach Anspruch 9, wobei die Expression des Genprodukts mit einer Produktionsausbeute bewirkt wird, die sich von der Produktionsausbeute, die erzielt wird, wenn der Bordetella-Stamm ein homologes Gen, das dieses Strukturgen und dessen eigenen nativen Promotor umfaßt, enthält, unterscheidet.

11. Verfahren nach Anspruch 9 oder 10, wobei die Transformation des Bordetella-Stamms bewirkt wird, indem das hybride Gen an den geeigneten Genorten in das Chromosom des Bordetella-Stamms durch homologe Rekombination integriert wird.

12. Verfahren nach einem der Ansprüche 9 bis 11, wobei der Bordetella-Stamm Bordetella pertussis ist.

13. Verfahren zur Expression eines Genprodukts durch einen transformierten Bordetella-Stamm, das umfaßt, den Bordetella-Stamm von Anspruch 1 zu kultivieren.

14. Verfahren nach Anspruch 13, wobei der Bordetella-Stamm Bordetella pertussis ist.

15. Verfahren nach den Ansprüchen 13 und 14, welches umfaßt, das Genprodukt zu isolieren und zu reinigen.

## Revendications

1. Souche de *Bordetella* caractérisée en ce qu'elle est transformée par un gène hybride de coqueluche comprenant un gène structural de la coqueluche fusionné au niveau d'un codon d'initiation ATG à un promoteur de coqueluche natif mais autologue, ledit promoteur étant le promoteur *TOX* et le gène structural de la coqueluche étant le gène *FHA* ou *PRN*, ledit promoteur étant le promoteur *FHA* et le gène structural de la coqueluche étant le gène *TOX* ou *PRN*, ou ledit promoteur étant le promoteur *PRN* et le gène structural de la coqueluche étant le gène *TOX* ou *FHA*.

2. Souche selon la revendication 1, la souche de *Bordetella* transformée par ledit gène hybride de coqueluche étant *Bordetella pertussis*.

3. Souche selon la revendication 2, ladite souche transformée produisant, par culture de ladite souche transformée, un produit génétique avec un rendement de production modifié par rapport au rendement de production obtenu avec la même souche de *Bordetella* contenant un gène homologue comprenant un gène structural de la coqueluche fusionné au niveau d'un codon d'initiation ATG à son propre promoteur de coqueluche natif.

4. Souche de *Bordetella* génétiquement modifiée caractérisée en ce que le gène *FHA* est supprimé et en ce qu'un gène hybride *FHAp*/*TOX* est inséré dans le génome pour donner au moins deux copies du gène *TOX* dans le génome afin d'obtenir des niveaux d'expression des protéines codées par le gène *TOX* présent dans ladite souche génétiquement modifiée augmentés comparativement à la souche naturelle non modifiée.

5. Souche de *B. pertussis PRN* ⁻ transformée par *FHAp*/*PRN* n° 1290-4, déposée auprès de l'ATCC sous le numéro d'entrée 55155, ou souche de *B. pertussis TOX* ⁻ transformée par *FHAp*/*TOX* (TOX) n° 390-59, construite comme décrit dans l'exemple 1, ou souche de *B. pertussis FHA* ⁻transformée par *FHAp*/*TOX* (FHA) n° 590-11, construite comme décrit dans l'exemple 2, ou souche de *B. pertussis FHA* ⁻ transformée par *TOXp*/*FHA* (FHA) n° 890-49, construite comme décrit dans l'exemple 3.

6. Souche *FHA*⁻ de *Bordetella pertussis* n° 390-101, ayant le numéro d'entrée ATCC 55157, ou souche *PRN* ⁻ de *Bordetella pertussis* n° 1090-108-3, ayant le numéro d'entrée ATCC 55156.

7. Plasmide S-3680-10 comprenant *FHAp*/*TOX* et des régions flanquantes *TOX* ayant la carte de restriction représentée dans la figure 1, ou plasmide S-3749-18 comprenant *FHAp*/*TOX* et des régions flanquante*s FHA* ayant la carte de restriction représentée dans la figure 2, ou plasmide S-3838-9 comprenant *TOXp*/*FHA* avec des régions flanquantes *FHA* ayant la carte de restriction représentée dans la figure 4, ou plasmide S-3982-7 comprenant *FHAp*/*PRN* avec des régions flanquantes *PRN* ayant la carte de restriction représentée dans la figure 6.

8. Vaccin à cellules entières contre *Bordetella pertussis* comprenant une forme tuée d'une souche transformée de *Bordetella pertussis* selon une quelconque des revendications 1 à 5 et un support physiologiquement acceptable de cette souche.

9. Méthode d'expression d'un produit génétique à partir d'une souche de *Bordetella* transformée, caractérisée en ce que (a) on transforme une souche de *Bordetella* par un gène hybride de la coqueluche comprenant un gène structural de la coqueluche codant pour un antigène de la coqueluche fusionné au niveau d'un codon d'initiation ATG à un promoteur de la coqueluche natif mais autologue, ledit promoteur étant le promoteur *TOX* et le gène structural de la coqueluche étant le gène *FHA* ou *PRN*, ledit promoteur étant le promoteur *FHA* et le gène structural de la coqueluche étant le gène *TOX* ou *PRN*, ou ledit promoteur étant le promoteur *PRN* et le gène structural de la coqueluche étant le gène *TOX* ou *FHA* et (b) on cultive ladite souche de *Bordetella* transformée pour obtenir l'expression dudit antigène de la coqueluche constituant ledit produit génétique à partir de ladite souche transformée.

10. Méthode selon la revendication 9, ladite expression dudit produit génétique s'effectuant avec un rendement de production différent du rendement de production atteint quand la souche de *Bordetella* contient un gène homologue comprenant ledit gène structural et son propre promoteur natif.

11. Méthode selon la revendication 9 ou 10, la transformation de ladite souche de *Bordetella* s'effectuant par intégration dudit gène hybride au niveau des loci appropriés dans le chromosome de la souche de *Bordetella* par recombinaison homologue.

12. Méthode selon une quelconque des revendications 9 à 11, dans laquelle ladite souche de *Bordetella* est *Bordetella pertussis*.

13. Méthode d'expression d'un produit génétique à partir d'une souche de *Bordetella* transformée, comprenant la culture de la souche de *Bordetella* de la revendication 1.

14. Méthode selon la revendication 13, dans laquelle ladite souche de *Bordetella* est *Bordetella pertussis*.

15. Méthode selon les revendications 13 et 14, incluant l'isolement et la purification du produit génétique.
